Europäisches Patentamt

European Patent Office

Office européen des brevets

(11) Veröffentlichungsnummer: **0 379 010**

**A1**

## (12) EUROPÄISCHE PATENTANMELDUNG

(21) Anmeldenummer: **90100218.8**

(51) Int. Cl.5: **A61B 7/02**

(22) Anmeldetag: **05.01.90**

(30) Priorität: **16.01.89 DE 8900402 U**

(43) Veröffentlichungstag der Anmeldung:
**25.07.90 Patentblatt 90/30**

(84) Benannte Vertragsstaaten:
**CH DE FR LI**

(71) Anmelder: **KIRCHNER & WILHELM GMBH & CO.**
**Eberhardstrasse 56**

**D-7144 Asperg 1(DE)**

(72) Erfinder: **Ulrich, Kirchner**
**Paulinenstrasse 25**
**D-7145 Markgröningen(DE)**

(74) Vertreter: **Schmid, Berthold**
**Patentanwälte Dipl.-Ing. B. Schmid Dr. Ing. G. Birn Falbenhennenstrasse 17**
**D-7000 Stuttgart 1(DE)**

(54) **Stethoskop.**

(57) Um ein Stethoskop bzw. dessen Stethoskopkopf oder Bruststück an die unterschiedlichen, bei der Benutzung des Stethoskops überlicherweise auftretenden Frequenzen oder Frequenzbereiche, insbesondere stufenlos anpassen zu können, läßt sich der lichte Durchtrittsquerschnitt für die Schallwellen von der Membrane 5 bzw. dem Tonaufnehmer zur Schallkammer 8 des Grundkörpers 1, der durch eine Ringnut 28 des Grundkörpers 1 und einen Deckel 2 begrenzt ist, von einem Maximalwert des wirksamen Durchströmquerschnitts bis zu einem Minimalwert oder theoretisch bis zum Wert Null verändern. Hierzu ist der Stethoskopkopf mit einer Art Drehschieber ausgebildet, wobei der Gehäusedeckel 2 das drehbare Schieberelement bildet. Dadurch ist es möglich, die Veränderung des wirksamen Durchströmquerschnitts bei aufliegender Membrane 5 vorzunehmen, ohne daß eine Relativbewegung zwischen Membrane und Körper des Patienten notwendig ist.

Fig. 1

## Stethoskop

Die Erfindung bezieht sich auf ein Stethoskop mit einem Tonaufnehmer und einer Gehäuse-Schallkammer, die mit einem Verbindungsschlauch zu einem Ohr-Bügel akustisch verbunden ist. Der Tonaufnehmer wird in bekannter Weise auf den Körper des Patienten aufgesetzt und er nimmt die vom Körper erzeugten akustischen Schallwellen auf. Durch das Gehäuse hindurch werden diese Schallwellen an den Verbindungsschlauch und letztlich an den Ohr-Bügel weitergeleitet. In Abhängigkeit von der jeweils abgehörten Stelle bzw. dem momentan untersuchten Organ entstehen Schallwellen unterschiedlicher Frequenz. Es ist leicht einzusehen, daß ein Stethoskop, welches nur einen Tonaufnehmer besitzt, im Grunde genommen nur für eine Frequenz oder einen begrenzten Frequenzbereich optimal ausgelegt ist. Aus diesem Grunde wurde bereits vorgeschlagen, ein Stethoskop mit zwei Tonaufnehmern auszustatten, die man wahlweise akustisch mit dem Verbindungsschlauch und damit dem Ohrbügel verbinden kann. Damit läßt sich eine gute Anpassung an zwei Frequenzen oder Frequenzbereiche vornehmen.

Es hat sich nun aber gezeigt, daß es wünschenswert wäre, wenn man ohne Veränderung von Stethoskopteilen eine Anpassung an mehr als zwei Frequenzen oder Frequenzbereich vornehmen könnte.

Die Aufgabe der Erfindung besteht infolgedessen darin, ein Stethoskop der eingangs genannten Art so weiterzubilden, daß es ohne Abänderung seiner einzelnen Teile leicht eine bestimmte Schallfrequenz oder einen Frequenzbereich angepaßt werden kann.

Zur Lösung dieser Aufgabe wird erfindungsgemäß vorgeschlagen, daß das Stethoskop gemäß dem Oberbegriff des Anspruchs 1 entsprechend dem kennzeichnenden Teil dieses Anspruchs ausgebildet ist. Die Schallkammer dieses Stethoskops ist über einen Auslaßdurchbruch fest an den Verbindungsschlauch angeschlossen. Weil sie wenigstens eine Schalleintrittsöffnung aufweist und der Tonaufnehmer mit mindestens einer Schallaustrittsöffnung versehen ist, kann man die oder eine Schallaustrittsöffnung der oder einer Schalleintrittsöffnung so zuordnen, daß sich zugeordnete Öffnungen teilweise überlappen oder der volle Durchströmquerschnitt zur Verfügung steht. Im Falle mehrerer Schalleintritts- und Schallaustrittsöffnung ist dafür zu sorgen, daß zumindest jeweils ein Durchlaß für die Schallwellen vom Tonaufnehmer zur Gehäuseschallkammer vorhanden ist und dieser in Abhängigkeit von der aufzunehmenden Frequenz seinen maximal großen oder auch nur einen verringerten wirksamen Querschnitt hat. Dies schließt selbstverständlich nicht aus, daß beispielsweise ein Querschnitt mit voller Durchgängigkeit und zusätzlich an anderer Stelle ein weiterer Querschnitt mit reduzierter Durchgängigkeit vorhanden sind.

Das Stethoskop kann nun werkseits auf einen ganz bestimmten Durchströmquerschnitt bzw. Gesamtdurchströmquerschnitt vom Tonaufnehmer zur Schallkammer eingestellt werden, so daß es für eine ganze bestimmte Frequenz oder einen bestimmten Frequenzbereich besonders vorteilhaft ist. Wenn jedoch gemäß einer Weiterbildung der Erfindung die Relativlage der Schalleintrittsöffnung gegenüber ihrer korrespondierenden Schallaustrittsöffnung veränderbar ist, was man durch Verstellen der letzteren gegenüber der ersteren oder auch umgekehrt erreichen kann und was beim Vorhandensein mehrerer Schalleintritts- und/oder Schallaustrittsöffnungen sinngemäß gilt, so kann man den wirksamen Strömungsquerschnitt vom Tonaufnehmer zur Schallkammer den Bedürfnissen bzw. dem jeweiligen Anwendungsfall entsprechend ändern. Zwekmäßigerweise handelt es sich um eine stufenlose Veränderung des Überströmquerschnitts.

Eine weitere Ausgestaltung der Erfindung ergibt sich aus Anspruch 3. Hierbei bilden der Gehäusedeckel und der Tonaufnehmer gewissermaßen einen Drehschieber, der vor oder gegebenenfalls auch während der Benutzung des Stethoskops verstellt werden kann, um dadurch eine optimale Anpassung an die jeweilige Untersuchung vornehmen zu können. Wenn man gemäß dieser Ausgestaltung den Gehäusedeckel gegenüber dem Tonaufnehmer drehbar ausbildet, so hat dies den Vorteil, daß der Tonaufnehmer während der Verstellung dieses Stethoskops auf dem Körper des Patienten verbleiben kann und während der Einstellung keine Relativbewegung zum Körper ausführt.

Eine bevorzugte Ausführungsform der Erfindung kennzeichnet sich dadurch, daß die Drehbewegung des Gehäusedeckels durch eine kreisbogenförmige Aufnahme und ein darin eingreifendes Anschlagglied begrenzt ist, wobei sich insbesondere die Aufnahme am Gehäusedeckel und das Anschlagglied am Tonaufnehmer befinden. Die Größe des Drehwinkels richtet sich in erster Linie nach der Größe der Schalleintritts- und Schallaustrittsöffnung. Er sollte so gewählt werden, daß ein Übergang von der vollständig freigegebenen Schalleintrittsöffnung bis etwa zur vollständig abgesperrten Schalleintrittsöffnung möglich ist. Falls mehrere Öffnungen in Drehrichtung nebeneinander angeordnet sind, so muß der Drehwinkel entsprechend größer gewählt werden, um dadurch von einer Öffnung auf die andere übergehen zu können.

Die beiden Drehendstellungen sind vorteilhafterweise mittels eines Schneppers und zweier Schneppaufnahmen fixierbar, wobei sich insbesondere das federbelastete Schneppergied am Tonaufnehmer und die Schnepperaufnahmen am Gehäusedeckel befinden.

Den drehbar gelagerten Gehäusedeckel kann man entweder mit einer Rändelung und/oder in Weiterbildung der Erfindung mit einer vorzugsweise radial abstehenden Drehhandhabe ausstatten.

Eine weitere Variante der Erfindung entnimmt man Anspruch 7. Bei einem Stethoskop üblicher Abmessungen und einer kreisförmigen Schalleintritts- sowie Schallaustrittsöffnung nimmt man für die größere Schallaustrittsöffnung einen Bohrungsdurchmesser in der Größenordnung von 1 Zentimeter, während man die kleinere Schallaustrittsöffnung in der Größenordnung von etwa einem Millimeter Durchmesser wählt. Dies sind allerdings bevorzugte Werte, von denen im Rahmen der Erfindung selbstverständlich jederzeit abgewichen werden kann.

Eine weitere bevorzugte Ausführungsform der Erfindung ergibt sich aus Anspruch 8. Wenn in einer ersten Ausgangsstellung die größere Schallaustrittsöffnung des Tonaufnehmers die Schalleintrittsöffnung des Gehäuses vollständig freigibt und der lichte Durchgangsquerschnitt durch Drehen des Gehäusedeckels stetig verkleinert wird, so kann man die Verhältnisse und Anordnungen so treffen, daß etwa dann, wenn ein minimaler wirksamer Durchtrittsquer schnitt vorhanden ist, ein Weiterdrehen des Gehäusedeckels in gleichem Drehsinne zu einer Freigabe der zweiten Eintrittsöffnung des Gehäusedeckels führt, bevor die erste Eintrittsöffnung vollständig geschlossen ist. Man kann auf diese quasi einen stufenlosen Übergang vom größten bis zum kleinsten wirksamen Durchströmquerschnitt erreichen.

Eine weitere Ausgestaltung der Erfindung besteht darin, daß zumindest die kleinere, vorzugsweise aber beide Schallaustrittsöffnungen, an ihrem vom Gehäusedeckel abgewandten Ende trichterartig erweitert sind. Der Schall wird dadurch besser zu den eigentlichen Durchbrüchen oder Bohrungen geleitet.

Der Schallaufnehmer ist in bevorzugter Weise als Membrankopf ausgebildet, d.h. er besteht in an sich bekannter Weise aus einem eine Schallaustrittsöffnung oder -öffnungen aufweisenden, insbesondere metallenen, plattenförmigen Grundkörper, einem Aufschraubring und einer dazwischen befindlichen, eingeklemmten Membrane.

Die Erfindung wird nachstehend anhand der Zeichnung näher erläutert. Hierbei stellen dar:

Fig. 1 Eine explosionsartige Darstellung der wesentlichsten Teile des Bruststücks oder Stethoskopkopfes, teilweise im Halbschnitt und teilweise in einem Vertikallängsmittelschnitt,

Fig. 2 eine Draufsicht auf den Gehäusedeckel in Pfeilrichtung A der Figur 1 gesehen,

Fig. 3 eine Draufsicht auf den plattenförmigen Grundkörper in Pfeilrichtung B der Fig. 1 gesehen.

Der Stethoskopkopf besteht im wesentlichen aus einem Schallgehäusegrundkörper 1, einem Gehäusedeckel 2, einem plattenförmigen Grundkörper 3, einer Befestigungsschraube 4, einer Membrane 5 sowie einem Aufschraubring 6. Am Schallgehäusegrundkörper 1 ist ein Verbindungsschlauch 7 angeschlossen, der in bekannter Weise als Doppelschlauch ausgebildet ist oder sich an seinem stetoskopkopffernen Ende gabelt, wobei beide Schlauchenden mit je einem Rohr des Ohrbügels verbunden sind. Vom Inneren der Schallkammer 8 erfolgt die akustische Verbindung mit dem Verbindungsschlauch 7 und dem nicht dargestellten, an sich bekannten Ohrbügel über eine Querbohrung 9 des Schallgehäuse-Grundkörpers 1.

Der Schallgehäusegrundkörper 1 besitzt einen zentrischen, absatzartig nach außen hin reduzierten Lagerzapfen 10, für den als gelochte Kreisscheibe ausgebildeten Gehäusedeckel 2. Der Durchmesser der zentrischen Bohrung 11 entspricht unter Berücksichtigung eines entsprechenden Lagerspiels etwa dem Außendurchmesser des freien Lagerzapfenendes mit reduziertem Durchmesser. Außerdem entnimmt man der Zeichnung, daß der Lagerzapfen 10 mit einer zentrischen Gewindebohrung 12 ausgestattet ist. In diese wird die

Befestigungsschraube 4 eingedreht, nachdem zuvor der Gehäusedeckel 2 und der plattenförmige Grundkörper am Schallgehäuse-Grundkörper 1 anmontiert worden sind. In axialer Richtung ist das Spiel so gewählt, daß sich bei festgezogener Schraube 4 der Gehäusedeckel 2 noch leicht im Sinne des Doppelpfeils 13 drehen läßt. Hierzu dient eine radial abstehende zapfenförmige Handhabe 14, die an ihrem Außenumfang vorzugsweise gerändelt ist.

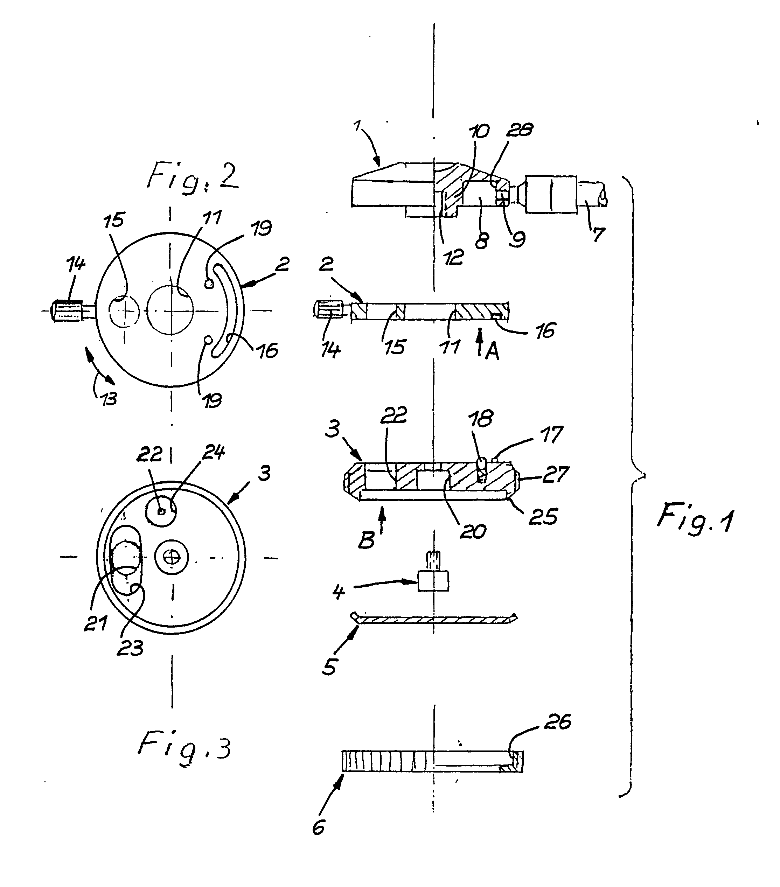

Wie Fig. 2 der Zeichnung zeigt, befindet sich seitlich der zentrischen Bohrung 11 am Gehäusedeckel 2 eine Schalleintrittsöffnung 15. Vorzugsweise um 180° versetzt hierzu ist im Bereich des Außenrandes am Gehäusedeckel 2 eine kreisbogenförmige Aufnahme 16 für ein Anschlagsglied 17 des plattenförmigen Grundkörpers 3 angebracht. Beide zusammen bilden eine Drehanschlagbegrenzung für den Gehäusedeckel 2. Außerdem können die beiden Drehendstellungen mittels eines Schneppers arretiert werden, wobei sich das federbelastete Schneppergied 18 bevorzugterweise am plattenförmigen Grundkörper 3 befindet, während die zugehörigen Schnepperaufnahmen 19 in die vom Schallgehäusegrundkörper 1 abgewandte Fläche des Gehäusedeckels eingearbeitet sind. Sowohl die Schnepperaufnahmen als auch die kreisbogenförmige Aufnahme 16 sind lediglich Vertiefungen, d.h. sie können nicht durchströmt werden. Der plattenförmige Grundkörper 3 ist mit einer zentrischen, im Durchmesser entsprechend der Schraube 4 abgesetzten Befestigungsbohrung 20 ausgestattet. Außerdem sind daran eine größere Schallaustrittsöffnung 21 sowie eine kleinere Schallaustrittsöffnung 22 angebracht. Es handelt sich um zwei Bohrungen unterschiedlichen Durchmessers. Beide gehen in eine gegen die Membrane 5 hin offene trichterartige Erweiterung 23 bzw. 24 über. Letztere ist kegelstumpfförmig, während erstere eher die Form eines beidseitig eingedrückten Kegelstumpfes hat. Außerdem ist am plattenförmigen Grundkörper 3 ein Auflagerand 25 für die Membrane 5 vorgesehen, welche mit Hilfe des Aufschraubrings 6 an diesen Rand 25 angepreßt wird. Der Aufschraubring besitzt in bekannter Weise ein Innengewinde 26, während beim plattenförmigen Grundkörper ein entsprechendes Außengewinde 27 angebracht ist.

Beim Ausführungsbeispiel sind die Schallaustrittsöffnungen 21 und 22 sowie die Schalleintrittsöffnung 15 so dimensioniert und angeordnet, daß bei vollständig freigegebener kleinerer Schallaustrittsöffnung 22 die größere Schallaustrittsöffnung 21 vom Gehäusedeckel 2 völlig überdeckt ist. Außerdem sind die Durchmesser der Schalldurchtrittsöffnungen 15 und 21 in etwa gleich groß.

**Ansprüche**

1. Stethoskop mit einem Tonaufnehmer und einer Gehäuse-Schallkammer (8), die mit einem Verbindungsschlauch (7) zu einem Ohrbügel akustisch verbunden ist, dadurch gekennzeichnet, daß die Schallkammer (8) mit wenigstens einer Schalleintrittsöffnung (15) und der Tonaufnehmer (3, 5, 6) mit mindestens einer Schallaustrittsöffnung (21, 22) versehen sind, wobei die oder zumindest eine Schallaustrittsöffnung (21, 22) der oder wenigstens einer Schalleintrittsöffnung (15) mindenstens teilweise überlappend zugeordnet oder zuordnenbar ist.

2. Stethoskop nach Anspruch 1, dadurch gekennzeichnet, daß die Relativlage der Schalleintrittsöffnung (15) gegenüber ihrer korrespondierenden Schallaustrittsöffnung (21, 22) veränderbar ist.

3. Stethoskop nach Anspruch 2, dadurch gekennzeichnet, daß die Gehäuse-Schallkammer (8) einen die Schalleintrittsöffnung (15) oder -öffnungen aufweisenden Gehäusedeckel (2) besitzt, der drehbar am Gehäusegrundkörper (1) gelagert ist, und daß sich der Gehäusedeckel (2) zwischen dem Grundkörper (1) und dem Tonaufnehmer (3, 5, 6) befindet, wobei letzterer drehfest mit dem Grund körper (1) verbunden ist.

4. Stethoskop nach Anspruch 3, dadurch gekennzeichnet, daß die Drehbewegung (13) des Gehäusedeckels (2) durch eine kreisbogenförmige Aufnahme (16) und ein darin eingreifendes Anschlagsglied (17) begrenzt ist, wobei sich insbesondere die Aufnahme (16) am Gehäusedeckel (2) und das Anschlagglied (17) am Tonaufnehmer (3, 5, 6) befinden.

5. Stethoskop nach Anspruch 4, dadurch gekennzeichnet, daß die beiden Drehendstellungen mittels eines Schneppers und zweier Schnepperaufnahmen (19) fixierbar sind, wobei sich insbesondere das federbelastete Schnepperglied (18) am Tonaufnehmer (30, 6) und die Schnepperaufnahme (19) am Gehäusedeckel (2) befinden.

6. Stethoskop nach wenigstens einem der Ansprüche 3 bis 5, dadurch gekennzeichnet, daß der Gehäusedeckel (2) mit einer vorzugsweise radial abstehenden Drehhandhabe (14) ausgestattet ist.

7. Stethoskop nach wenigstens einem der Ansprüche 3 bis 6, dadurch gekennzeichnet, daß der Gehäusedeckel (2) mit einer Schalleintrittsöffnung (15) und der Schallaufnehmer (3, 5, 6) mit zwei Schallaustrittsöffnungen (21, 22) unterschiedlichen Querschnitts ausgestattet sind, wobei der lichte Querschnitt der größeren Schallaustrittsöffnung (21) etwa dem lichten Querschnitt der Schalleintrittsöffnung (15) entspricht.

8. Stethoskop nach Anspruch 7, dadurch gekennzeichnet, daß die beiden Schallaustrittsöffnungen (21, 22) des Schallaufnehmers (3, 5, 6) in Umfangsrichtung des Gehäusedeckels (2) derart versetzt angeordnet sind, daß die kleinere (22) etwa dann mit der Schalleintrittsöffnung (15) akustisch verbunden ist, wenn die größere (21) vom Gehäusedeckel (2) zumindest weitgehend verschlossen ist.

9. Stethoskop nach Anspruch 7 oder 8, dadurch gekennzeichnet, daß zumindest die kleinere, vorzugsweise aber beide Schallaustrittsöffnungen (21, 22) an ihrem vom Gehäusedeckel (2) abgewandten Ende trichterartig (23, 24) erweitert sind.

10. Stethoskop nach wenigstens einem der vorhergehenden Ansprüche, dadurch gekennzeichnet, daß der Schallaufnehmer (3, 4, 6) als Membrankopf ausgebildet ist.

Fig.2

Fig.3

Fig.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | US-A-4072822 (YAMADA)<br>* Figuren 1-11 *<br>* Spalte 1, Zeile 57 - Spalte 3, Zeile 46 * | 1 | A61B7/02 |
| A | | 2, 3 | |
| | --- | | |
| X | DE-U-8513039 (KIRCHNER & WILHELM)<br>* Figuren *<br>* Seite 7, Zeile 3 - Seite 9, Zeile 25 * | 1 | |
| A | | 3-5, 10 | |
| | --- | | |
| A | US-A-3951230 (LITTMAN)<br>* Figuren 1-3 *<br>* Spalte 2, Zeile 25 - Spalte 3, Zeile 26 * | 1 | |
| | --- | | |
| A | FR-A-2396376 (LIESSE)<br>* Figuren 7-10 *<br>* Seite 3, Zeile 15 - Seite 5, Zeile 21 * | 1 | |
| | ----- | | |

RECHERCHIERTE SACHGEBIETE (Int. Cl.5 )

A61B

Der vorliegende Recherchenbericht wurde für alle Patentansprüche erstellt

| Recherchenort | Abschlußdatum der Recherche | Prüfer |
|---|---|---|
| DEN HAAG | 03 MAI 1990 | CHEN A.H. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
..............................................................................
& : Mitglied der gleichen Patentfamilie, übereinstimmendes
    Dokument

EPO FORM 1503 03.82 (P0403)